# EUROPÄISCHE PATENTSCHRIFT

(11) **EP 0 356 858 B1**
(45) Veröffentlichungstag und Bekanntmachung des Hinweises auf die Patenterteilung: **19.10.1994**
(21) Anmeldenummer: 89115357.9
(22) Anmeldetag: 19.08.1989
(51) Int. Cl.: C07C 35/08, C07C 29/86

(54) **Verfahren zur Abtrennung von Cyclohexanol**
Process for the separation of cyclohexanol
Procédé de séparation du cyclohexanol

(30) Priorität: 27.08.1988 DE 3829142
(43) Veröffentlichungstag der Anmeldung: 07.03.1990
(73) Patentinhaber: BASF Aktiengesellschaft, 67063 Ludwigshafen (DE)
(72) Erfinder: Gosch, Hans-Juergen, Dr., D-6702 Bad Duerkheim (DE); Fischer, Rolf, Dr., D-6900 Heidelberg (DE)

(56) Entgegenhaltungen:
- EP-A- 0 227 931
- EP-A- 0 285 911

## Beschreibung

Aus der europäischen Patentanmeldung 123 713 ist ein Verfahren bekannt, bei dem man Cyclohexanol durch Hydratisieren von Cyclohexen bei einer Temperatur von 50 bis 200°C in 5 bis 80 Gew.%igen Lösungen von aromatischen Sulfonsäuren in Wasser erhält. Um Korrosion zu verhindern, wird die Umsetzung z.B. in Gegenwart von Heteropolysäuren, Salzen oder Oxiden des Molybdäns, Wolframs oder Vanadins durchgeführt. Das durch Hydratisierung von Cyclohexen gebildete Cyclohexanol löst sich überwiegend in der aromatische Sulfonsäuren enthaltenden wäßrigen Phase und wird auch durch überschüssiges Cyclohexen nur zum geringen Teil aus dieser abgetrennt.

Entsprechend der japanischen Offenlegungsschrift 103 033/1987 werden zur Extraktion von Cyclohexanol aus aromatischen Sulfonsäuren enthaltenden wäßrigen Lösungen aliphatische, cycloaliphatische und aromatische Kohlenwassserstoffe empfohlen. Hierbei sind jedoch erhebliche Mengen Extraktionsmittel erforderlich, um eine wirkungsvolle Extraktion zu erreichen. Dies ist technisch sehr aufwendig.

Nach einem in der europäischen Patentanmeldung 227 931 beschriebenen Verfahren wird die Hydratisierung von Cyclohexen in wäßrigen Lösungen von aromatischen Sulfonsäuren, die mindestens eine Hydroxylgruppe am aromatischen Ring haben, durchgeführt und anschließend mit aromatischen Kohlenwasserstoffen extrahiert. Hierbei gelingt es zwar die Menge an Extraktionsmittel zu senken, nachteilig ist jedoch, daß durch die Verwendung der Hydroxylgruppen enthaltenden aromatischen Sulfonsäuren die Ausbeute und Selektivität an Cyclohexanol erheblich vermindert wird.

Die ältere europäische Anmeldung 285 911, die in den Ländern BE, DE, FR, GB, IT und NL gültig ist, beschreibt die Extraktion von Cyclohexanol aus aromatische Sulfonsäuren enthaltenden wäßrigen Lösungen mit Lösungen aus Kohlenwasserstoffen und Phenol.

Es war deshalb die technische Aufgabe gestellt, die Abtrennung von Cyclohexanol aus wäßrigen aromatischen Sulfonsäurelösungen, wie sie bei der Hydratisierung von Cyclohexen in Gegenwart von aromatischen Sulfonsäuren anfallen, zu verbessern.

Diese Aufgabe wird gelöst in einem Verfahren zur Abtrennung von Cyclohexanol aus solches und aromatisches Sulfonsäuren enthaltenden wäßrigen Lösungen durch Extraktion, wobei man als Extraktionsmittel Gemische aus
a) mindestens einem bei Extraktionsbedingungen inerten flüssigen aliphatischen, cycloaliphatischen oder aromatischen Kohlenwasserstoff, Halogenkohlenwasserstoff oder Ether und
b) mindestens einem Phenol
verwendet.

Das neue Verfahren hat den Vorteil, daß die anzuwendende Menge an Extraktionsmittel erheblich vermindert wird und ein besseres Resultat bei der Extraktion erzielt wird. Dies gilt auch bei der Verwendung von keine Hydroxylgruppen enthaltenden Sulfonsäuren als Hydratisierungsmittel bei der Herstellung von Cyclohexanol, wodurch zusätzlich die Ausbeute an Cyclohexanol gesteigert wird.

Erfindungsgemäß geht man von wäßrigen Lösungen aus, die Cyclohexanol und aromatische Sulfonsäuren gelöst enthalten. Solche Lösungen erhält man bei der Hydratisierung von Cyclohexen zu Cyclohexanol, wobei man Cyclohexen in wäßriger Lösung, die 5 bis 80 Gew.% aromatische Sulfonsäuren insbesondere Benzol- oder Naphthalinsulfonsäuren, die substituiert sein können, wie Benzolsulfonsäure, Toluolsulfonsäure oder Naphthalinsulfonsäure ferner Dodecylbenzolsulfonsäure enthalten bei einer Temperatur von 50 bis 200°C, insbesondere 70 bis 150°C unter einem Druck von 1 bis 10 bar umsetzt. Vorteilhaft werden zusätzlich Molybdänsäure oder deren Salze, Vanadinoxid oder Vanadate in einer Menge z.B. von 0,001 bis 5 Gew.%, bezogen auf die aromatische Sulfonsäure mitverwendet. Darüber hinaus ist es auch empfehlenswert, Heteropolysäuren wie Phosphormolybdänsäure, Phosporwolframsäure, Phosphormolybdänwolframsäure oder Phosphormolybdänvanadinsäure mitzuverwenden. Geeignete Verfahren werden beispielsweise beschrieben in der europäischen Patentschrift 123 713 oder der europäischen Patentanmeldung 206 631. Aus dem so erhaltenen Reaktionsgemisch scheidet sich überschüssiges Cyclohexen, das geringere Mengen Cyclohexanol enthält, als organische Phase ab. Eine typische Zusammensetzung der erhaltenen wäßrigen Phase ist beispielsweise 5 bis 10 Gew.% Cyclohexanol, 40 bis 70 Gew.% aromatische Sulfonsäuren und 20. bis 50 Gew.% Wasser. Die Cyclohexenphase kann z.B. durch Dekantieren abgetrennt und getrennt aufgearbeitet werden. Für die Extraktion ist es jedoch nicht unbedingt erforderlich, Cyclohexen abzutrennen, da es als zusätzliches Extraktionsmittel wie nachfolgend beschrieben, mitverwendet werden kann.

Die so erhaltene wäßrige Lösung wird mit einem Extraktionsmittelgemisch aus
a) mindestens einem bei Extraktionsbedingungen inerten flüssigen aliphatischen, cycloaliphatischen oder aromatischem Kohlenwasserstoff Halogenkohlenwasserstoff oder Ether und
b) mindestens einem Phenol extrahiert.

Geeignete Kohlenwasserstoffe haben in der Regel 6 bis 12 Kohlenstoffatome geeignet sind beispielsweise Benzol, Toluol, Cyclohexen, Decalin oder Tetralin. Geeignete Halogenkohlenwasserstoffe haben in der Regel 1 oder 2 Kohlenstoffatome wie z.B. Fluor-, Chlor-, Bromkohlenwasserstoffe, insbesondere Chlorkohlenwasserstoffe beispielsweise Methylenchlorid, Chloroform, Dichlorethan, Dichlorethylen oder Tetrachlorkohlenstoff. Geeignete Ether haben in der Regel 6 bis 12 Kohlenstoffatome. Geeignet sind beispielsweise Diphenylether oder Cyclohexylether. Besonders geeignet sind Cyclohexylether wie Dicyclohexylether, Cyclohexylmethylether, Cyclohexylethylether, Cyclohexyl-n-propylether, Cyclohexyl-i-propylether, Cyclohexyl-n-butylether, Cyclohexyl-i-butylether oder Phenylcyclohexylether.

Geeignete Phenole sind beispielsweise ein-, zwei- oder dreikernige Phenole mit bis zu 3 Hydroxylgruppen, die zusätzlich Alkylreste mit 1 bis 15 Kohlenstoffatomen, Alkoxyreste mit 1 bis 4 Kohlenstoffatomen, Halogenatome wie Chlor oder Brom als Substituenten haben können. Geeignete Phenole sind beispielsweise Phenol o-, m-, p-Alkylphenole wie Kresole, Xylenole, Trimethylphenole, Ethylphenole, Tertiärbutylphenole, Tertiäramylphenole, Nonylphenole, 4-Methyl-2,6-ditertiärbutylphenol, 2-Tertiärbutylmethylphenol, 2,5-Ditertiärbutylhydrophenol, Phenylphenol, Chlorphenole, Hydrochinon, Resorcin, Brenzcatechin, Pyrogallol, Hydrochinonmonomethylether oder deren Gemische. Besondere Bedeutung haben einkernige Phenole mit bis zu 2 Hydroxylgruppen und bis zu 3 Alkylgruppen, jeweils mit bis zu 15 Kohlenstoffatomen als Substituenten erlangt.

In der Regel enthält das Extraktionsmittel 0,1 bis 90 Gew.%, mindestens eines Phenols. Besonders bewährt haben sich Mengen von 5 bis 50 Gew.%, insbesondere 10 bis 40 Gew.%.

Auf einen Volumenteil der zu extrahierenden, Cyclohexanol und aromatische Sulfonsäuren enthaltenden wäßrigen Lösung wendet man zweckmäßig 0,1 bis 50 Vol.-teile, insbesondere 1 bis 10 Vol.-teile des Extraktionsmittelgemisches an. Die Extraktion wird im allgemeinen bei einer Temperatur von 0 bis 200°C vorteilhaft von 10 bis 100°C, insbesondere 10 bis 50°C durchgeführt.

Die Extraktion der Cyclohexanol und aromatische Sulfonsäuren enthaltenden wäßrigen Lösung kann absatzweise durchgeführt werden, indem man die wäßrige Lösung intensiv mit dem Phenol enthaltenden Extraktionsmittel vermischt und anschließend die Phasen trennt. Vorteilhaft wird die Extraktion kontinuierlich im Gegenstrom in geeigneten Extraktionskolonnen wie Rührscheibenkolonnen, Pulsationskolonnen oder ein oder mehrstufigen Mixer-Settler-Vorrichtungen durchgeführt. Die anfallende aromatische Sulfonsäure enthaltende wäßrige Phase wird wiederum für die Hydratisierung von Cyclohexen verwendet, während die Cyclohexanol enthaltende Extraktionsmittelphase durch Destillation aufgearbeitet wird. Kleine Mengen an Phenolen, die bei der Phasentrennung in die aromatische Sulfonsäure enthaltende Wasserphase gelangen, stören bei der Hydratisierung nicht. Falls in dem Cyclohexanol enthaltenden Extrakt geringe Mengen an aromatischen Sulfonsäuren enthalten sind, lassen sich diese durch Waschen mit wenig Wasser leicht entfernen und werden zweckmäßig der wäßrigen Lösung für die Hydratisierung zugefügt.

Bei einer bevorzugten Ausführungsform des Extraktionsverfahrens verwendet man ein Extraktionsmittel, das höher als Cyclohexanol siedet und einen für eine wirtschaftliche Trennung ausreichenden Siedepunktabstand zu Cyclohexanol z.B. >10°C hat. In diesem Fall muß lediglich das Cyclohexanol durch Destillation vom Extraktionsmittel abgetrennt werden. Das als Sumpfphase anfallende Extraktionsmittel wird zweckmäßig wiederum in die Extraktionsstufe zurückgeführt. In einer bevorzugten Ausführungsform wird die Extraktion unter Mitverwendung der bei der Hydratisierung anfallenden überschüssigen Cyclohexenphase durchgeführt. Bei der anschließenden Aufarbeitung erhält man somit zunächst eine Cyclohexenfraktion die wiederum für die Hydratisierung verwendet wird, eine Cyclohexanolfraktion und als Sumpf das Extraktionsmittel.

Cyclohexanol, das nach dem Verfahren der Erfindung erhältlich ist, eignet sich zur Herstellung von Cyclohexanon, einem wichtigen Ausgangsstoff für Caprolactam.

Das Verfahren nach der Erfindung sei in folgenden Beispielen veranschaulicht. Die Gewichtsteile verhalten sich den Vol.-Teilen wie kg zu Litern.

### Beispiel 1

In einem Gefäß wurden 17,4 Vol.-Teile einer wäßrigen Lösung aus 54 Gew.-Teilen p-Toluolsulfonsäure, 10 Gew.-Teilen Cyclohexanol und 36 Gew.-Teilen Wasser mit 22,9 Vol.-Teilen einer Lösung von 15 Gew.% 4-Nonylphenol in Toluol für 5 Minuten bei Raumtemperatur auf einer Schüttelmaschine intensiv gemischt. Nach Trennung der Phasen wurde gaschromatographisch ermittelt, daß 45 Gew.% des eingesetzten Cyclohexanols durch die Phenol-Toluol-Lösung aus der wäßrigen Phase extrahiert worden waren.

### Beispiele 2 - 9

Die Extraktion wurde wie in Beispiel 1 beschrieben durchgeführt, jedoch wurden als Extraktionsmittel verschiedene Phenole jeweils in Toluol gelöst verwendet. Die Ergebnisse sind in Tabelle 1 wiedergegeben:

**Tabelle 1**

| Beispiel | Phenol | Konz. Phenol [Gew.%] | Extrahiertes Cyclohexanol [Gew.%] |
|---|---|---|---|
| 2 | Phenol | 15 | 22.7 |
| 3 | 4-Methylphenol | 15 | 22.8 |
| 4 | 4-tert.Butylphenol | 10 | 29.1 |
| 5 | 4-tert.Amylphenol | 15 | 30.2 |
| 6 | 2-tert.Butyl-4-methylphenol | 15 | 41.6 |
| 7 | 2,6-Di-tert.butylphenol | 10 | 16.6 |
| 8 | 2,6-Di-tert.butyl-4-methylphenol | 10 | 17.0 |
| 9 | 2,5-Di-tert.butylhydrochinon | 2 | 19.1 |

### Beispiele 10 - 12

Die Extraktion wurde wie in Beispiel 1 beschrieben durchgeführt, jedoch wurde eine wäßrige Lösung von 64 Gew.-Teilen p-Toluolsulfonsäure, 10 Gew.-Teilen Cyclohexanol und 26 Gew.-Teilen Wasser sowie eine 15 gew.%ige Lösung verschiedener Phenole in Toluol als Extraktionsmittel verwendet. Die Ergebnisse sind in Tabelle 2 zusammengefaßt.

**Tabelle 2**

| Beispiel | Phenol | Konz. Phenol [Gew.%] | Extrahiertes Cyclohexanol [Gew.%] |
|---|---|---|---|
| 10 | 4-tert.Amylphenol | 15 | 16.1 |
| 11 | 4-Nonylphenol | 15 | 28.1 |
| 12 | 2-tert.Butyl-4-methylphenol | 15 | 25.1 |

### Beispiele 13 - 18

Die Durchführung der Extraktion erfolgte wie in Beispiel 1 beschrieben, jedoch wurde jeweils eine 15 gew.%ige Lösung folgender Phenole in verschiedenen Kohlenwasserstoffen als Extraktionsmittel verwendet. Die Ergebnisse sind in Tabelle 3 zusammengefaßt:

**Tabelle 3**

| Beispiel | Kohlenwasserstoffe wie Ether | Extrahiertes Cyclohexanol [Gew.%] | |
|---|---|---|---|
| | | 2tB4m¹⁾ | 4Nonyl²⁾ |
| 13 | Cyclohexen | 47.0 | - |
| 14 | Toluol | 41.6 | 44.5 |
| 15 | Tetralin | 41.0 | 45.1 |
| 16 | Decalin | 34.8 | 47.6 |
| 17 | 4-tert.Butyltoluol | 38.9 | - |
| 18 | Dicyclohexylether | 35.6 | - |

| | | | |
|---|---|---|---|
| ¹⁾ 2tB4m = 2-tert.Butyl-4-methylphenol | | | |
| ²⁾ 4Nonyl = 4-Nonylphenol | | | |

### Beispiele 19 - 22

Die Durchführung der Extraktion erfolgte wie in Beispiel 1 beschrieben, nur wurde als wäßrige Phase eine Lösung aus 64 Gew.-Teilen p-Toluolsulfonsäure, 10 Gew.-Teilen Cyclohexanol und 26 Gew.-Teilen Wasser verwendet und eine 15 gew.%ige Lösung der angegebenen Phenole in verschiedenen Kohlenwasserstoffen als Extraktionsmittel verwendet. Die Ergebnisse sind in Tabelle 4 aufgeführt:

**Tabelle 4**

| Beispiel | Extraktionsmittel | Extrahiertes Cyclohexanol [Gew.%] | |
|---|---|---|---|
| | | 2tB4m | 4Nonyl |
| 19 | Toluol | 24.5 | 28.1 |
| 20 | Tetralin | 25.8 | 25.6 |
| 21 | Decalin | 18.6 | 31.7 |
| 22 | 4-tert.Butyltoluol | 23.4 | 32.0 |

### Beispiele 23 - 27

Zur kontinuierlichen Durchführung der Extraktion wurde eine wäßrige Lösung aus 54 Gew.-Teilen p-Toluolsulfonsäure, 10 Gew.-Teilen Cyclohexanol und 36 Gew.-Teilen Wasser in einer 5-stufigen Mixer-Settler-Apparatur mit einer Lösung von 15 Gew.% 2-tert.Butyl-4-methylphenol in Tetralin kontinuierlich bei Raumtemperatur extrahiert. Bei den einzelnen Beispielen wurde jeweils das Verhältnis von wäßriger Lösung zu Extraktionsmittel variiert. Die Verweilzeit in der Apparatur betrug zwischen 1.3 und 2.2 h. Die Menge des extrahierten Cyclohexanols wurde gaschromatographisch ermittelt. Die Ergebnisse sind in Tabelle 5 zusammengefaßt:

**Tabelle 5**

| Beispiel | Vol.-Verhältnis wäßrige Lösung zu Extraktionsmittel | Verweilzeit [h] | Extrahiertes Cyclohexanol [Gew.%] |
|---|---|---|---|
| 23 | 1 : 7 | 1.5 | 99 |
| 24 | 1 : 3.7 | 1.3 | 99 |
| 25 | 1 : 2.6 | 1.3 | 98 |
| 26 | 1 : 2 | 1.4 | 86 |
| 27 | 1 : 1 | 2.2 | 46 |

### Beispiel 28

In einem Druckbehälter aus Glas wurden pro Stunde 320 Gew.-Teile 60%ige wäßrige p-Toluolsulfonsäure und 32 Gew.-Teile Cyclohexen bei 118°C und 5 bar Druck miteinander umgesetzt. Das zweiphasige Gemisch wurde während der Reaktion im Reaktor intensiv gerührt (800 Upm). Nach Verlassen des Reaktors wurde in einem Phasenscheider das nicht umgesetzte Cyclohexen abetrennt und die wäßrige p-Toluolsulfonsäure-Phase mit dem darin enthaltenen Cyclohexanol in eine 5-stufige Mixer-Settler-Apparatur überführt. In der Mixer-Settler-Apparatur wurde das weitgehend vom Cyclohexen befreite Reaktionsgemisch kontinuierlich mit einer 15 gew.%igen Lösung von 2-tert.Butyl-4-methylphenol in Tetralin extrahiert. Das Volumenverhältnis von wäßriger Lösung zu Extraktionsmittel betrug 1 : 2.3 und die Verweilzeit 1.3 h. Nach dem Trennen von wäßriger und organischer Phase wurde die organische Phase, welche nach Passieren der Mixer-Settler-Apparatur das Cyclohexanol enthielt, einem Fallfilmverdampfer mit Füllkörperkolonne zugeführt. Die wäßrige p-Toluolsulfonsäure-Lösung wurde nach Zusatz des bei der Reaktion verbrauchten Wassers in die Hydratisierungsstufe zurückgegeben. Bei einer Verdampfertemperatur von 115°C, einem Druck von 30 mbar und einem Rücklaufverhältnis von 1 : 5 wurde am Kopf der Kolonne ein Destillat erhalten, das aus 64 Gew.% Cyclohexanol, 12 Gew.% Wasser, 14 Gew.% Cyclohexen und 9 Gew.% Tetralin bestand. Am Sumpf des Verdampfers konnte eine vom Cyclohexanol befreite Phenol-Tetralin-Lösung entnommen werden, welche erneut zur Extraktion des Cyclohexanols verwendet wurde.

Der Versuch lief über einen Zeitraum von 100 Stunden. Während dieser Zeit wurde ein durchschnittlicher Cyclohexenumsatz von 53 Mol.%, bei einer Cyclohexanolselektivität von 97 % erreicht. Die durchschnittliche Cyclohexanolausbeute betrug 51,5 Mol.%.

### Vergleichsbeispiele 1 - 5

Die Extraktion wurde wie im Beispiel 1 angegeben durchgeführt, jedoch wurden zur Extraktion jeweils die reinen Kohlenwasserstoffe verwendet. Die Ergebnisse sind in Tabelle 6 wiedergegeben:

**Tabelle 6**

| Vergleichsbeispiel | Extraktionsmittel | Extrahiertes Cyclohexanol [Gew.%] |
|---|---|---|
| 1 | Cyclohexen | 13.7 |
| 2 | Toluol | 16.4 |
| 3 | Tetralin | 14.4 |
| 4 | Decalin | 5.2 |
| 5 | 4-tert.Butyltoluol | 11.6 |

### Vergleichsbeispiele 6 - 9

Die Extraktion wurde wie im Vergleichsbeispiel 1 angegeben durchgeführt, jedoch wurde als wäßrige Phase eine Lösung aus 64 Gew.% p-Toluolsulfonsäure, 10 Gew.% Cyclohexanol und 26 Gew.% Wasser verwendet. Die Ergebnisse sind in Tabelle 7 zusammengefaßt:

**Tabelle 7**

| Vergleichsbeispiel | Extraktionsmittel | Extrahiertes Cyclohexanol [Gew.%] |
|---|---|---|
| 6 | Toluol | 9.8 |
| 7 | Tetralin | 9.2 |
| 8 | Decalin | 2.9 |
| 9 | 4-tert. Butyltoluol | 6.7 |

### Vergleichsbeispiel 10

Die Extraktion wurde kontinuierlich, wie in den Beispielen 23 - 27 beschrieben, durchgeführt. An Stelle einer Phenol-Tetralin-Lösung wurde reines Toluol zur Extraktion verwendet. Das Volumenverhältnis von wäßriger Phase zu Toluol betrug 1 : 5 und die Verweilzeit 1.5 h. Unter diesen Bedingungen wurden 62 % des Cyclohexanols aus der wäßrigen Phase extrahiert.

## Patentansprüche (Patentansprüche für folgende(n) Vertragsstaat(en): BE, DE, FR, GB, IT, NL)

1. Verfahren zur Abtrennung von Cyclohexanol aus solches und aromatische Sulfonsäuren enthaltenden wäßrigen Lösungen durch Extraktion, dadurch gekennzeichnet, daß man als Extraktionsmittel Gemische aus
a) mindestens einem bei Extraktionsbedingungen inerten flüssigen Halogenkohlenwasserstoff oder Ether und
b) mindestens einem Phenol
verwendet.

2. Verfahren nach Anspruch 1, dadurch gekennzeichnet, daß man einkernige Phenole mit bis zu 2 Hydroxylgruppen und mit bis zu 3 Alkylresten mit jeweils bis zu 15 Kohlenstoffatomen als Substituenten verwendet.

3. Verfahren nach den Ansprüchen 1 und 2, dadurch gekennzeichnet, daß man Cyclohexylether verwendet.

4. Verfahren nach den Ansprüchen 1 bis 3, dadurch gekennzeichnet, daß man je Volumenteil wäßrige Lösung 1 bis 10 Volumenteile Extraktionsmittel verwendet.

5. Verfahren nach den Ansprüchen 1 bis 4, dadurch gekennzeichnet, daß das Extraktionsmittel 5 bis 50 Gew.% mindestens eines Phenols enthält.

6. Verfahren nach den Ansprüchen 1 bis 5, dadurch gekennzeichnet, daß das Extraktionsmittel einen höheren Siedepunkt als Cyclohexanol hat.

7. Verfahren nach den Ansprüchen 1 bis 6, dadurch gekennzeichnet, daß man die Extraktion bei einer Temperatur von 10 bis 100°C durchführt.

8. Verfahren nach den Ansprüchen 1 bis 7, dadurch gekennzeichnet, daß man eine wäßrige Lösung, die durch Hydratisierung von Cyclohexen in einer wäßrigen Lösung einer aromatischen Sulfonsäure erhalten wurde, ohne Abtrennung des überschüssigen Cyclohexens für die Extraktion verwendet.

## Patentansprüche (Patentansprüche für folgende(n) Vertragsstaat(en): CH, ES, LI)

1. Verfahren zur Abtrennung von Cyclohexanol aus solches und aromatische Sulfonsäuren enthaltenden wäßrigen Lösungen durch Extraktion, dadurch gekennzeichnet, daß man als Extraktionsmittel Gemische aus
a) mindestens einem bei Extraktionsbedingungen inerten flüssigen aliphatischen, cycloaliphatischen oder aromatischen Kohlenwasserstoff, Halogenkohlenwasserstoff oder Ether und
b) mindestens einem Phenol
verwendet.

2. Verfahren nach Anspruch 1, dadurch gekennzeichnet, daß man einkernige Phenole mit bis zu 2 Hydroxylgruppen und mit bis zu 3 Alkylresten mit jeweils bis zu 15 Kohlenstoffatomen als Substituenten verwendet.

3. Verfahren nach den Ansprüchen 1 und 2, dadurch gekennzeichnet, daß man Cyclohexylether verwendet.

4. Verfahren nach den Ansprüchen 1 bis 3, dadurch gekennzeichnet, daß man je Volumenteil wäßrige Lösung 1 bis 10 Volumenteile Extraktionsmittel verwendet.

5. Verfahren nach den Ansprüchen 1 bis 4, dadurch gekennzeichnet, daß das Extraktionsmittel 5 bis 50 Gew.% mindestens eines Phenols enthält.

6. Verfahren nach den Ansprüchen 1 bis 5, dadurch gekennzeichnet, daß das Extraktionsmittel einen höheren Siedepunkt als Cyclohexanol hat.

7. Verfahren nach den Ansprüchen 1 bis 6, dadurch gekennzeichnet, daß man die Extraktion bei einer Temperatur von 10 bis 100°C durchführt.

8. Verfahren nach den Ansprüchen 1 bis 7, dadurch gekennzeichnet, daß man eine wäßrige Lösung, die durch Hydratisierung von Cyclohexen in einer wäßrigen Lösung einer aromatischen Sulfonsäure erhalten wurde, ohne Abtrennung des überschüssigen Cyclohexens für die Extraktion verwendet.

## Claims (Claims for the following Contracting State(s): BE, DE, FR, GB, IT, NL)

1. A process for removing cyclohexanol from an aqueous solution containing it and an aromatic sulfonic acid by extraction, which comprises using as the extractant a mixture of
a) one or more liquid (hydro)halocarbons or ethers which are inert under extraction conditions and
b) one or more phenols.

2. A process as claimed in claim 1, wherein a monocyclic phenol having up to 2 hydroxyl groups and up to 3 alkyl groups, each of up to 15 carbon atoms, as substituents is used.

3. A process as claimed in claim 1 or 2, wherein a cyclohexyl ether is used.

4. A process as claimed in any of claims 1 to 3, wherein from 1 to 10 parts by volume of extractant are used per part by volume of aqueous solution.

5. A process as claimed in any of claims 1 to 4, wherein the extractant contains from 5 to 50% by weight of one or more phenols.

6. A process as claimed in any of claims 1 to 5, wherein the extractant has a higher boiling point than cyclohexanol.

7. A process as claimed in any of claims 1 to 6, wherein the extraction is carried out at from 10 to 100°C.

8. A process as claimed in any of claims 1 to 7, wherein an aqueous solution obtained by hydration of cyclohexene in an aqueous solution of an aromatic sulfonic acid is used for the extraction without removal of the excess cyclohexene.

## Claims (Claims for the following Contracting State(s): CH, ES, LI)

1. A process for removing cyclohexanol from an aqueous solution containing it and an aromatic sulfonic acid by extraction, which comprises using as the extractant a mixture of
a) one or more liquid aliphatic, cycloaliphatic or aromatic hydrocarbons, (hydro)halocarbons or ethers which are inert under extraction conditions and
b) one or more phenols.

2. A process as claimed in claim 1, wherein a monocyclic phenol having up to 2 hydroxyl groups and up to 3 alkyl groups, each of up to 15 carbon atoms, as substituents is used.

3. A process as claimed in claim 1 or 2, wherein a cyclohexyl ether is used.

4. A process as claimed in any of claims 1 to 3, wherein from 1 to 10 parts by volume of extractant are used per part by volume of aqueous solution.

5. A process as claimed in any of claims 1 to 4, wherein the extractant contains from 5 to 50% by weight of one or more phenols.

6. A process as claimed in any of claims 1 to 5, wherein the extractant has a higher boiling point than cyclohexanol.

7. A process as claimed in any of claims 1 to 6, wherein the extraction is carried out at from 10 to 100°C.

8. A process as claimed in any of claims 1 to 7, wherein an aqueous solution obtained by hydration of cyclohexene in an aqueous solution of an aromatic sulfonic acid is used for the extraction without removal of the excess cyclohexene.

## Revendications (Revendications pour l'(les) Etat(s) contractant(s) suivant(s): BE, DE, FR, GB, IT, NL)

1. Procédé de séparation de cyclohexanol à partir de solutions aqueuses contenant celui-ci et des acides sulfoniques aromatiques, par extraction, caractérisé en ce qu'on utilise comme milieu d'extraction des mélanges de
a) au moins un hydrocarbure halogéné ou un éther liquide inerte dans les conditions d'extraction et
b) au moins un phénol.

2. Procédé selon la revendication 1, caractérisé en ce qu'on utilise des phénols monocycliques avec jusqu'à 2 groupements hydroxy et jusqu'à 3 restes alkyle ayant chacun jusqu'à 15 atomes de carbone comme substituants.

3. Procédé selon les revendications 1 et 2, caractérisé en ce qu'on utilise de l'éther cyclohexylique.

4. Procédé selon les revendications 1 à 3, caractérisé en ce qu'on utilise de 1 à 10 parties en volume de milieu d'extraction par partie en volume de solution aqueuse.

5. Procédé selon les revendications 1 à 4, caractérisé en ce que le milieu d'extraction contient 5 à 50% d'au moins un phénol.

6. Procédé selon les revendications 1 à 5, caractérisé en ce que le milieu d'extraction a un point d'ébullition supérieur à celui du cyclohexanol.

7. Procédé selon les revendications 1 à 6, caractérisé en ce qu'on mène l'extraction à une température de 10 à 100°C.

8. Procédé selon les revendications 1 à 7, caractérisé en ce qu'on utilise une solution aqueuse qui a été obtenue par hydratation de cyclohexène dans une solution aqueuse d'un acide sulfonique aromatique, sans séparation du cyclohexène en excès pour l'extraction.

## Revendications (Revendications pour l'(les) Etat(s) contractant(s) suivant(s): CH, ES, LI)

1. Procédé de séparation de cyclohexanol à partir de solutions aqueuses contenant celui-ci et des acides sulfoniques aromatiques, par extraction, caractérisé en ce qu'on utilise comme milieu d'extraction des mélanges de
a) au moins un hydrocarbure aliphatique, cycloaliphatique ou aromatique, un hydrocarbure halogéné ou un éther liquide inerte dans les conditions d'extraction et
b) au moins un phénol.

2. Procédé selon la revendication 1, caractérisé en ce qu'on utilise des phénols monocycliques avec jusqu'à 2 groupements hydroxy et jusqu'à 3 restes alkyle ayant chacun jusqu'à 15 atomes de carbone comme substituants.

3. Procédé selon les revendications 1 et 2, caractérisé en ce qu'on utilise de l'éther cyclohexylique.

4. Procédé selon les revendications 1 à 3, caractérisé en ce qu'on utilise de 1 à 10 parties en volume de milieu d'extraction par partie en volume de solution aqueuse.

5. Procédé selon les revendications 1 à 4, caractérisé en ce que le milieu d'extraction contient 5 à 50% d'au moins un phénol.

6. Procédé selon les revendications 1 à 5, caractérisé en ce que le milieu d'extraction a un point d'ébullition supérieur à celui du cyclohexanol.

7. Procédé selon les revendications 1 à 6, caractérisé en ce qu'on mène l'extraction à une température de 10 à 100°C.

8. Procédé selon les revendications 1 à 7, caractérisé en ce qu'on utilise une solution aqueuse qui a été obtenue par hydratation de cyclohexène dans une solution aqueuse d'un acide sulfonique aromqtique, sans séparation du cyclohexène en excès pour l'extraction.
